# EUROPEAN PATENT SPECIFICATION

(11) **EP 4 384 540 B1**
(45) Date of publication and mention of the grant of the patent: **06.08.2025**
(21) Application number: 22760952.6
(22) Date of filing: 04.08.2022
(51) Int. Cl.: C07K 14/435, A61P 19/08, A61K 8/64, A61K 6/69, A61C 8/02, A61C 8/00, A61Q 19/00, A61K 38/00

(54) **BIOMIMETIC PEPTIDES AND THEIR USE IN BONE REGENERATION**
BIOMIMETISCHE PEPTIDE UND IHRE VERWENDUNG BEI DER KNOCHENREGENERATION
PEPTIDES BIOMIMÉTIQUES ET LEUR UTILISATION DANS LA RÉGÉNÉRATION OSSEUSE

(30) Priority: 09.08.2021 IT 202100021557
(43) Date of publication of application: 19.06.2024
(73) Proprietor: Klisbio S.r.l., 20091 Bresso (MI) (IT)
(72) Inventor: BIAGIOTTI, Marco, 20900 MONZA (IT); FREDDI, Giuliano, 20030 Senago (IT); ALESSANDRINO, Antonio, 22100 Como (IT); SIRONI, Maurizio, 20066 Melzo (IT); PIERACCINI, Stefano, 20137 Milano (IT); DAPIAGGI, Federico, 4310 Rheinfelden (CH)
(74) Representative: Zaccaro, Elisabetta
(86) International application number: PCT/EP2022/071898
(87) International publication number: WO 2023/016904

(56) References cited:
- CN-A- 102 703 488
- KR-A- 20110 121 401
- US-A1- 2010 317 587
- US-B2- 7 193 038
- DU MINGZU ET AL: "Recent advances in biomedical engineering of nano-hydroxyapatite including dentistry, cancer treatment and bone repair", COMPOSITES PART B, ELSEVIER, AMSTERDAM, NL, vol. 215, 16 March 2021 (2021-03-16), XP086577858, ISSN: 1359-8368, [retrieved on 20210316], DOI: 10.1016/J.COMPOSITESB.2021.108790

## Description

### FIELD OF THE INVENTION

The invention concerns biomimetic peptides having a common pentapeptide sequence, which allow bone regeneration by activating the hydroxyapatite (Hap) deposition process. Their uses as a medicament, and specifically in the field of bone, dental (dentin and enamel) and periodontal regeneration, and in the functionalization of prostheses, screws, fixings, inserts or supports made of metal, ceramic, or natural or synthetic polymeric material are also described. Further described are a pharmaceutical composition and a cosmetic composition comprising such peptides, the use as a medicament, in bone, dental and periodontal regeneration, its cosmetic use and its use in the functionalization of prostheses, screws, fixings, inserts or supports made of metal, ceramic, or natural or synthetic polymeric material are further described.

### STATE OF THE ART

The assembly of hydroxyapatite (HAp) and proteins is a crucial process underlying bone and tooth formation in vertebrates. The human body is able to produce these hierarchically structured organic-inorganic hybrid composites using organic molecules as regulators of HAp deposition.

HAp [Ca₁₀(PO₄)₆(OH)₂], a crystalline form of calcium phosphate (CaP), is the main component of bone, dentin and enamel. In these hard tissues, HAp, in combination with various types of proteins, forms highly organised hierarchical structures with unique morphological, structural and mechanical properties.

Bone is formed by 65-70% of mineral components, mainly HAp, and by 30-35% of organic molecules. The organic component comprises mainly type I collagen, various other non-collagenous type proteins and glycosaminoglycans.

The organisation of type I collagen is important for the deposition of HAp in the bone tissue. Two α-1 chains and one α-2 chain of type I collagen constitute the fundamental unit of collagen fibrils, the tropocollagen. The tropocollagen units form a highly aligned structure, and the gap regions between the fibrils, called "hole zones" and having a high charge density, are the critical sites where the nucleation of the mineral component takes place.

Non-collagen type proteins such as bone sialoprotein, osteonectin, osteopontin, osteocalcin and dentin matrix proteins bind to tropocollagen and control the mineralization of HAp. These proteins include a high density of acidic amino acid residues in their sequences, which have a high affinity for Ca²⁺ ions.

Tooth enamel is constituted by mineral components for more than 95% by weight and for less than 1% by organic components. Instead of by type I collagen present in the bone, the most important role for the mineralization of HAp in enamel is played by amelogenin, with the contribution of other proteins (ameloblastin, amelin, sheathelin, enamelin, and matrix metalloproteinase 20). Nanospheres of self-assembled amelogenin and mineral pre-nucleation groups form composite nanoparticles that then are assembled into linear chains and parallel matrices, leading to the formation of bundles of elongated crystals of enamel.

The repair of bone and dental defects and/or gaps can be performed by autograft (use of bone derived from the patient himself, taken from other body sites) or allograft (bone material derived from a cadaver). In both cases, often insurmountable problems may arise due to a lack of harvesting sites (autograft) or immunogenicity problems (allograft). For these reasons, the research and development of new materials for bone and dental repair is a highly topical issue. Having the principles of tissue engineering as a reference, tissue and organ regeneration can be achieved through the implantation of engineered constructs. The success of an engineered construct depends on the use of an appropriate combination of scaffolds, stimulating factors and possibly cells.

The understanding of the mechanisms underlying the mineralization of HAp with organic molecules has been a source of inspiration for the design of new functional materials. Many researchers have studied the formation of organic hybrids of HAp using organic molecules, mainly proteins, under biological conditions that mimic the biomineralization process.

As far as proteins are concerned, those related to the biomineralization of HAp *in vivo* have always been considered as ideal candidates for the production of protein-HAp hybrids. In fact, the mineralization of HAp with collagen and amelogenin, which are the organic components that regulate HAp deposition in bone and teeth, has been studied very thoroughly. Non-collagen type proteins and proteins other than amelogenin that help control HAp deposition in bone and enamel have also been used for the preparation of protein-HAp hybrids. All these organic-inorganic hybrids, used as scaffolds for bone regeneration, in the presence or absence of cells, have provided positive results by promoting the osteogenic differentiation of cells and improving the formation of new bone and integration.

The proteins used to generate organic hybrids of HAp are not limited to those related to bone and tooth mineralization. Silk proteins have been used as model proteins for the nucleation and deposition of HAp due to their high biocompatibility and to the mechanical and structural properties.

In addition to proteins, other natural macromolecules such as polysaccharides (e.g. chitin, chitosan, chondroitin sulphate) have aroused some interest as substrates capable of inducing and regulating HAp precipitation. Likewise, bacterial cellulose nanofibers have been used as a model capable of reproducing collagen-based structures to induce HAp deposition.

A very interesting alternative that has recently emerged is the use of biomimetic peptides as substrates capable of activating HAp nucleation if included within scaffolds for bone regeneration. Peptides can be easily produced both by direct synthesis of the sequences of interest and by genetic engineering methods. The sequences of interest can be designed *in silico* or derived from those of the active domains of the extracellular matrix proteins and of the large family of proteins directly involved in processes of *in vivo* bone mineralization (e.g. type I collagen, non-collagenous proteins, amelogenin, etc.), and then used to functionalize scaffolds for bone regeneration and/or directly to prepare peptide-HAp hybrids.

These biomimetic peptides may perform various functions within the matrix for bone regeneration: they may have osteoconductive activity and favour HAp nucleation and deposition, they may have osteoinductive activity and thus favour adhesion of the cells, their proliferation and differentiation in osteoblastic lines, and they could also be involved in the processes of neovascularization, which represent a crucial step for *in vivo* bone regeneration. Depending on the function performed, the biomimetic peptides can be incorporated into the scaffold in various positions (on the surface, in the mass) and in free or bound form, depending on whether or not their diffusion into the environment surrounding the implant is required.

The particular interest in the development of new materials for bone, periodontal and dental repair, together with the difficulties encountered in the case of autografts or allografts, are at the basis of the need to identify new approaches for the repair of defects in the field of bone regeneration.

The object of the present invention is therefore to provide novel biomimetic peptides that allow bone regeneration by activating and accelerating HAp nucleation phenomena, which do not show the disadvantages found for different peptides or proteins known for the same application. In particular, proteins belonging to the family of BMP (bone morphogenic protein) have several disadvantages linked to the extreme *in vivo* reactivity and the high cost of production. Two different members of this family have been commercially exploited: BMP-2 and BMP-7. The products containing BMP-7, after having received regulatory approvals only for use in very limited areas, were withdrawn from the market following the emergence of serious doubts about their safety; in fact, cases of various side effects have been reported, ranging from simple irritations up to serious infections that required repair surgery interventions. The products based on BMP-2 (in particular its recombinant version rH-BMP-2) are instead currently on the market and represent the standard of care for a certain number of applications, often competing with the use of autologous bone. Over time, however, a fairly large number of studies have highlighted many side effects, some even quite serious, which can be directly correlated to the use of this peptide and not identified in the first clinical trials; among the main ones we can mention: osteolysis, urological problems, emergence of pain and, in the case of higher dosages, an increase in the risk of developing tumours [E.J. Carragee et al. The Spine Journal 11 (2011) 471-491]. In addition, the products containing rH-BMP-2 have an extremely high cost. As far as peptides are concerned, instead, a sequence derived from type 1 collagen is currently used in the clinic; this sequence, known as P-15, has a very high ability to stimulate cell adhesion [H. Nguyen et al. Biochemical and Biophysical Research Communications 311 (2003) 179-186]; however, to be effective in promoting bone tissue formation, it must be bound to a preformed support, usually bone matrix, that provides the attracted cells with a favourable environment in which to develop and differentiate. KR 2011 0121401 A discloses a bone graft material to rapidly regenerate a damaged and lost part of bone by applying the bone graft material to the damaged and lost part of bone. Said bone graft material includes silk fibroin peptide as an active ingredient, wherein the silk fibroin peptide has average molecular weight of 100-4,000 Da.

### SUMMARY OF THE INVENTION

The invention is defined in the appended set of claims.

### DESCRIPTION OF THE DRAWINGS

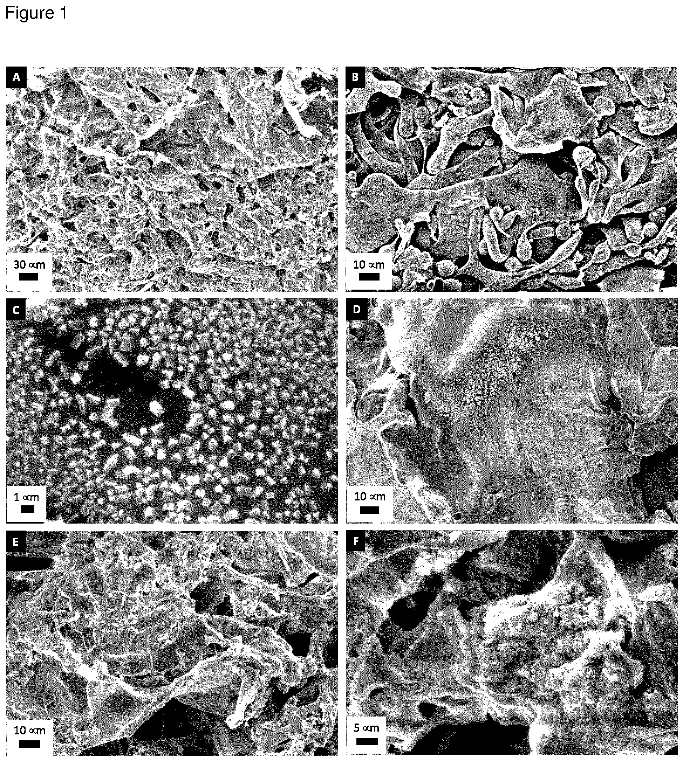

The invention will now be described in detail and with reference to the following accompanying Figures. Figure 1: SEM images of fibroin foams. A: Peptide-free fibroin foam, incubated in SBF for 14 days. B: Fibroin foam functionalized with the biomimetic peptide SEQ ID NO:3, incubated in SBF for 14 days. C: Higher magnification detail of image B, showing the morphology of the mineral deposits. D: Fibroin foam functionalized with the biomimetic peptide SEQ ID NO:4, incubated in SBF for 14 days. E: Fibroin foam functionalized with the biomimetic peptide SEQ ID NO:2, incubated in SBF for 14 days. F: Higher magnification detail of image E, showing the accumulation of the mineral deposits.

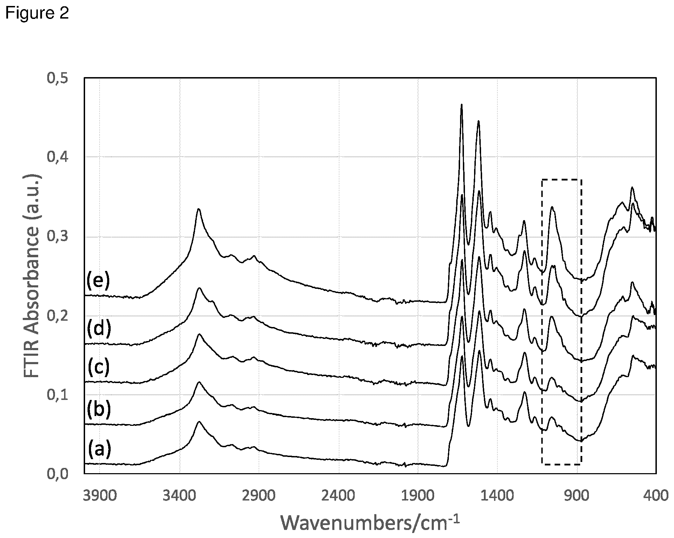

Figure 2: IR spectra of fibroin foams. (a) Peptide-free fibroin foam, not incubated in SBF. (b) Peptide-free fibroin foam, incubated in SBF for 14 days. (c) Fibroin foam functionalized with the biomimetic peptide SEQ ID NO:3, incubated in SBF for 14 days. (d) Fibroin foam functionalized with the biomimetic peptide SEQ ID NO:4, incubated in SBF for 14 days. (e) Fibroin foam functionalized with the biomimetic peptide SEQ ID NO:2, incubated in SBF for 14 days. The area enclosed in the dashed rectangle identifies the spectral region 1100-900cm⁻¹ characteristic of the stretching vibrations of phosphate and carbonate groups.

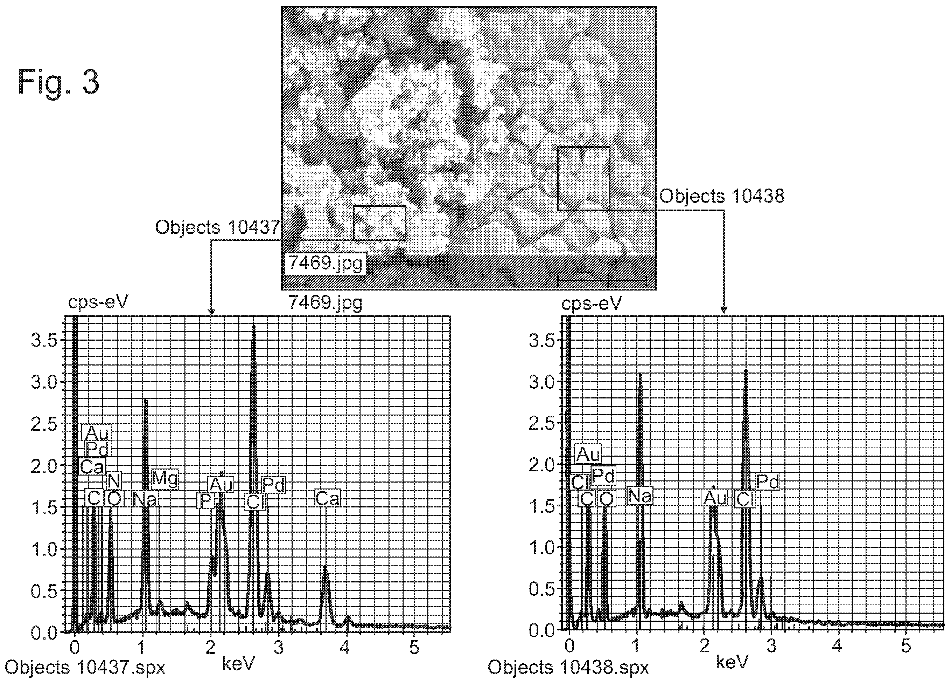

Figure 3: SEM/EDX spectra of fibroin foam functionalized with the biomimetic peptide SEQ ID NO:4 incubated in SBF for 14 days. The SEM image at the top shows a zone of the sample in which the two crystalline forms are present: on the right large-sized and regular-shaped crystals prevail (Objects 10438), on the left the crystalline aggregates consisting of the union of small-sized crystals are evident (Objects 10437). The chemical composition of the crystals "Objects 10438" is dominated by the presence of the elements sodium (Na) and chlorine (CI), while calcium (Ca) and phosphorus (P) are completely absent. In contrast, the aggregates "Object 10437" have intense signals of calcium (Ca) and phosphorus (P). The signals relative to the elements carbon (C), oxygen (O), nitrogen (N), gold (Au) and palladium (Pd), which are common to both spectra, reflect the organic nature of the fibroin foam (C, O, N) and the method of sample preparation (Au/Pd sputtering).

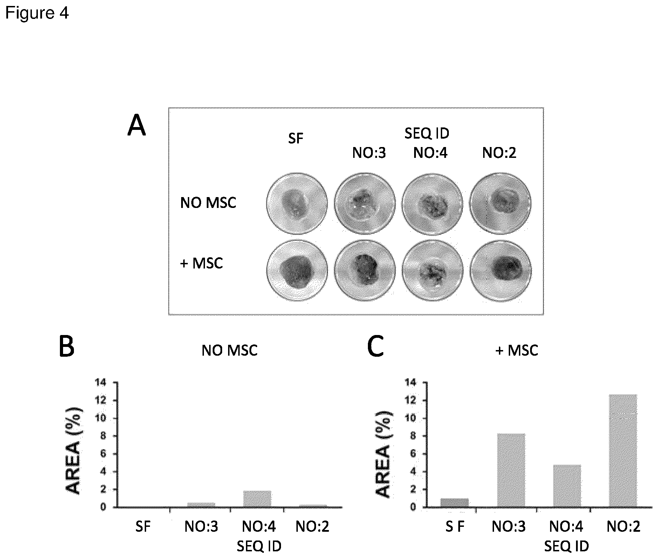

Figure 4: Fibroin foams non-functionalized (SF) and functionalized with the biomimetic peptides (SEQ ID NO:2; SEQ ID NO:3; SEQ ID NO:4), incubated in the absence (NO MSC) or in the presence (+ MSC) of human mesenchymal stem cells were maintained in a complete medium for 7 days. Subsequently, the foams were decellularized and stained with von Kossa staining to highlight calcium deposits (A). The percentage area covered by von Kossa staining was calculated using the ImageJ software and reported in graphs B and C. The amount of deposited calcium is always higher in fibroin foams functionalized with the biomimetic peptides than in non-functionalized foam, both in the absence (B) and in the presence (C) of MSC cells.

### DETAILED DESCRIPTION OF THE INVENTION

The present invention concerns the identification of novel biomimetic peptides of a synthetic nature, which have the characteristic of allowing bone regeneration by activating and accelerating the processes of HAp nucleation and deposition.

The biomimetic peptides consist of the general formula (I):
Xaa1-Ser-Gly-Tyr-Glu-Tyr-Xaa2 (SEQ ID NO: 5) wherein:
- when Xaa1 is Val-Asn-Gly-Gly-Tyr (SEQ ID NO:6),
   Xaa2 is Ala-Trp-Ser-Ser-Glu-Ser-Asp-Phe (SEQ ID NO:7)
      or
   Xaa2 is Ala-Trp;
- when Xaa1 is absent,
   Xaa2 is Ala-Trp-Ser-Ser-Glu-Ser-Asp-Phe (SEQ ID NO:7);
   and
- when Xaa1 is Gly-Pro-Tyr-Val-Ala-His-Gly-Gly-Tyr (SEQ ID NO:8),
   Xaa2 is absent.

All the peptides described herein have the pentapeptide sequence of SEQ ID NO:5 in common.

Advantageously in a preferred embodiment the peptides are chosen from the group consisting of:
SEQ ID NO:1 Val-Asn-Gly-Gly-Tyr-Ser-Gly-Tyr-Glu-Tyr-Ala-Trp-Ser-Ser-Glu-Ser-Asp-Phe,
SEQ ID NO:2 Ser-Gly-Tyr-Glu-Tyr-Ala-Trp-Ser-Ser-Glu-Ser-Asp-Phe,
SEQ ID NO:3 Gly-Pro-Tyr-Val-Ala-His-Gly-Gly-Tyr-Ser-Gly-Tyr-Glu-Tyr, and
SEQ ID NO:4 Val-Asn-Gly-Gly-Tyr-Ser-Gly-Tyr-Glu-Tyr-Ala-Trp.

All the peptides of SEQ ID NO:1-4 have the amino acid *core* of SEQ ID NO:5 in common. Without being bound to any theory, it would seem that it is precisely this *core* of five amino acids (pentapeptide) that allows the peptides the ability of bone regeneration by activating and accelerating nucleation phenomena of the HAp deposition process. These peptides were identified by the inventors through tests during which the peptides described in US7193038, known to facilitate cell adhesion and proliferation, are added to silk fibroin matrices.

The matrices (in particular electrospun surfaces, gels, pastes and foams) added with peptides according to the present invention have favoured cell proliferation and a rapid initiation of the mineralization processes with deposition of hydroxyapatite precursors even on the functionalized matrices incubated in environments favourable to bone growth both in the presence and in total absence of cells.

As a confirmation that this result was attributable to the presence of the peptides used for functionalization, experiments were conducted in the same environment but on non-functionalized matrices and, as expected, there was no (significant) nucleation of mineral components.

A second aspect of the present invention concerns the use as a medicament of the peptides having the general formula (I): Xaa1-Ser-Gly-Tyr-Glu-Tyr-Xaa2 (SEQ ID NO: 5)
wherein:
- when Xaa1 is Val-Asn-Gly-Gly-Tyr (SEQ ID NO:6),
   Xaa2 is Ala-Trp-Ser-Ser-Glu-Ser-Asp-Phe (SEQ ID NO:7)
      or
   Xaa2 is Ala-Trp;
- when Xaa1 is absent,
   Xaa2 is Ala-Trp-Ser-Ser-Glu-Ser-Asp-Phe (SEQ ID NO:7);
   and
- when Xaa1 is Gly-Pro-Tyr-Val-Ala-His-Gly-Gly-Tyr (SEQ ID NO:8),
   Xaa2 is absent.

In yet another aspect, the use of the peptides, having general formula (I) according to the present invention, in bone, dental (dentin and enamel) and periodontal regeneration as a substrate for HAp nucleation is described.

In a preferred embodiment, said bone, dental and periodontal regeneration is in the treatment of a pathology of the bone, dental or periodontal tissue or is required by a particular surgical technique.

In an even more preferred embodiment, said pathology of the bone, dental or periodontal tissue is chosen from the group comprising: fractures, bone gaps, sarcoma, dentinal hypersensitivity, pyorrhea, while the surgical technique may comprise posterolateral spinal fusion, anterior cervical dissectomy with fusion, posterior lumbar intervertebral fusion, transforaminal lumbar intervertebral fusion, orthopaedic endoprosthesis implantation, maxillary sinus lift, revision of orthopaedic endoprosthesis, vertebroplasty, dental implantation.

In a fourth aspect, the present invention describes a composition comprising one or more peptides, the same or different from each other, their salts, and excipients or additives acceptable in the biomedical, cosmetic or pharmaceutical field.

The composition of the invention may further comprise hyaluronic acid and/or their salts, chitosan, alginic acid, silk fibroin, propylene glycol, propylene glycol alginate, poloxamers, chondroitin sulfate, collagen, gelatin, elastin, polylactic acid (PLA), poly(lactic-co-glycolic) acid (PLGA), polyglycolic acid (PGA), polyvinyl alcohol (PVA), polycaprolactone (PCL), bioglasses, hydroxyapatite and correlated compounds, calcium salts, decellularized bone matrix, pectin, sericin, cellulose and their derivatives, fibrin and their combinations.

In a preferred embodiment, the pharmaceutical composition comprising one or more peptides, the same or different from each other, their salts and pharmacologically acceptable excipients is in the form of a gel, paste, membrane, fabric, foam, putty, powder, grains, compact solid, film or is contained/adsorbed within micellar or particle elements, fibers, nanofibers, hollow fibers, hollow nanofibers, membranes, ceramics, nanotubes, porous and/or trabecular metal structures, textile products, nanobubbles, sol-gels, combinations of these systems and composites containing these systems and their combinations. This composition in the form of gel or paste can be a toothpaste.

In a further preferred embodiment, the cosmetic composition comprising one or more peptides, the same or different from each other, their salts and excipients acceptable for the cosmetic formulation is in the form of gel, paste, membrane, fabric, foam, putty, powder, grains, compact solid, film or is contained/adsorbed within micellar or particle elements, fibers, nanofibers, hollow fibers, hollow nanofibers, membranes, ceramics, nanotubes, porous and/or trabecular metal structures, textile products, nanobubbles, sol-gels, combinations of these systems and composites containing these systems and their combinations. This composition in the form of gel or paste can be a toothpaste.

In yet another aspect, the present invention describes the use of the composition in bone, dental and periodontal regeneration.

The peptides of general formula (I):
Xaa1-Ser-Gly-Tyr-Glu-Tyr-Xaa2 (SEQ ID NO: 5) wherein:
- when Xaa1 is Val-Asn-Gly-Gly-Tyr (SEQ ID NO:6),
   Xaa2 is Ala-Trp-Ser-Ser-Glu-Ser-Asp-Phe (SEQ ID NO:7)
      or
   Xaa2 is Ala-Trp;
- when Xaa1 is absent,
   Xaa2 is Ala-Trp-Ser-Ser-Glu-Ser-Asp-Phe (SEQ ID NO:7);
   and
- when Xaa1 is Gly-Pro-Tyr-Val-Ala-His-Gly-Gly-Tyr (SEQ ID NO:8),
   Xaa2 is absent,
and described by means of the sequences SEQ ID NO:1-4 and used for the functionalization of silk fibroin matrices as described above.

The use of the composition according to the present invention as a medicament is also described.

In yet another aspect, the present invention describes the use of the pharmaceutical or cosmetic composition comprising the peptides described herein, for the surface functionalization of prostheses, screws, fixings, inserts or supports made of metal, ceramic or natural or synthetic polymeric material.

The present invention further describes the use of the peptides of the invention, for the surface functionalization of prostheses, screws, fixings, inserts or supports made of metal, ceramic or natural or synthetic polymeric material. In a further embodiment, the cosmetic use of the composition is described, comprising the peptides having the general formula (I):
Xaa1-Ser-Gly-Tyr-Glu-Tyr-Xaa2 (SEQ ID NO: 5) described by the present invention.

The following examples of embodiments of the present invention are reported below by way of illustration.

### EXAMPLES

### Example 1:

### Synthesis of the biomimetic peptides

A number of peptides in which the pentapeptide of SEQ ID NO:5 is present have been synthesized. The most promising sequences, and which have shown the ability of inducing a high degree of mineralization with deposition of hydroxyapatite precursors are:

SEQ ID NO:1 Val-Asn-Gly-Gly-Tyr-Ser-Gly-Tyr-Glu-Tyr-Ala-Trp-Ser-Ser-Glu-Ser-Asp-
Phe,

SEQ ID NO:2 Ser-Gly-Tyr-Glu-Tyr-Ala-Trp-Ser-Ser-Glu-Ser-Asp-Phe,
SEQ ID NO:3 Gly-Pro-Tyr-Val-Ala-His-Gly-Gly-Tyr-Ser-Gly-Tyr-Glu-Tyr, and
SEQ ID NO:4 Val-Asn-Gly-Gly-Tyr-Ser-Gly-Tyr-Glu-Tyr-Ala-Trp.

The peptides were synthesized following a solid phase peptide synthesis protocol with the aid of microwaves. A Wang resin was used as a support. Each coupling was made by working with an excess of 5 times over the initial loading of the resin and N,N' - Diisopropylcarbodiimide (DIC) (0.5 M in Dimethylformamide (DMF)) and the coupling agent Oxyma Pure^{®} (1 M in DMF) were used as coupling agents. The cleavage from the resin was carried out using a mixture of Phenol, triisopropylsilane (TIS), thioanisole (TAN), H₂O and trifluoroacetic acid (TFA) (4/4/4/2/86). The sequences were purified by semi-preparative RP-HPLC

### Example 2:

### Preparation of fibroin foams functionalized with the biomimetic peptides

The matrices of fibroin functionalized with the biomimetic peptides were prepared in the form of lyophilized foam, adopting the following procedure:
a) for the preparation of the aqueous fibroin solution, 5 g of degummed silk were added with 50 ml of an aqueous solution of Lithium Bromide 9.3 M; the mixture was incubated in a thermostatic bath at 60°C, for 3 hours; at the end of the incubation period, the solution was filtered and diluted with 100 ml of distilled water, transferred to a dialysis bag and dialysed for 2 days against distilled water until the salt was completely removed; the aqueous solution of fibroin thus obtained was recovered from the dialysis bag and filtered before being added with the biomimetic peptides;
b) for the preparation of the aqueous solutions of biomimetic peptides, 50 mg of peptide were solubilized with 2.5 ml of distilled water;
c) for the preparation of the fibroin solution containing biomimetic peptides, 10 ml of aqueous solution of fibroin were added with 2.5 ml of aqueous solution of biomimetic peptide; for the preparation of the fibroin solution without biomimetic peptides (blank) 10 ml of fibroin aqueous solution were added with 2.5 ml of water;
d) for the preparation of the lyophilized foams, 0.1 ml of the solution (c) were taken with a micropipette, transferred into a container of corresponding volume and frozen at -20°C for 24 hours; subsequently, the containers were placed in a lyophilizer and allowed to lyophilize for 48 hours, until the ice was completely removed by sublimation; after lyophilization the foams were consolidated by immersion in methanol and allowed to dry at room temperature.

The fibroin foam samples thus obtained were stored in a desiccator at room temperature until their subsequent use.

### Example 3:

### Incubation of fibroin foams functionalized with the biomimetic peptides in a favourable environment for bone growth (in the absence of cells)

The solution in which the biomimetic peptide-functionalized foams were incubated was prepared according to the method of Kokubo and Takadama (Kokubo T., Takadama H. How useful is SBF in predicting in vivo bone bioactivity? Biomaterials 2006 27(15): 2907-2915). The solution will henceforth be identified by the acronym SBF (Simulated Body Fluid).

The incubation of the foams in SBF was performed under the following experimental conditions:
- foam weight: 4 mg
- volume of the SBF solution: 40 ml
- temperature: 37°C
- maximum incubation duration: 14 days
- change of SBF solution: every 3 days

At the end of the incubation period, the foams were withdrawn, rinsed gently with distilled water and allowed to dry at room temperature before subsequent analyses.

### Example 4:

### Morphological characterization by scanning electron microscopy (SEM) of the fibroin foams incubated in SBF

The fibroin foam samples were fixed on 1 cm diameter SEM specimen holders (stubs) using double-sided adhesive tape. The stubs were then sputter-coated by exposure in gold/palladium plasma (sputter-coater Desk IV, Denton Vacuum, LLC) and analysed with a scanning electron microscope (Zeiss EVO MA10). The results obtained are reported in Figure 1.

All the samples examined, non-functionalized fibroin foam (blank) and fibroin foams functionalized with the biomimetic peptides, have a surface deposition of material with a polyhedral crystalline-like structure, which appears much brighter than the polymeric material in the background that constitutes the foam. These characteristics (regular structure and high intensity of response upon exposure to the electronic beam) are typical of inorganic materials.

The density of the inorganic deposit is significantly higher in the functionalized fibroin foam samples than in the blank (non-functionalized fibroin foam).

### Example 5:

### Characterization by infrared spectroscopy (ATR-FTIR) of the fibroin foams incubated in SBF

The fibroin foam samples were analysed by infrared spectroscopy with an ALPHA FTIR spectrometer (Bruker) equipped with an ATR Platinum Diamond accessory, in the wavelength number range 4000-400 cm⁻¹, at a resolution of 4 cm⁻¹. The spectra obtained are reported in Figure 2.

By comparing the spectra of the samples incubated in SBF (blank and foams functionalized with the biomimetic peptides) with the reference sample (fibroin foam not incubated in SBF), it can be observed that the incubated samples exhibit IR bands in the spectral range 1100-900 cm⁻¹ that are absent in the reference. These bands are attributed to the stretching vibration of groups such as phosphate and carbonate. The presence of these IR bands is therefore attributable to the incubation process in SBF. All the sponge samples incubated in SBF, both the blank and those functionalized with the biomimetic peptides, show these IR bands. However, it is interesting to observe that the intensity of the bands is clearly higher in the samples functionalized with biomimetic peptides, while it remains at a baseline level in the blank, in accordance with the morphological observations reported in Example 4.

### Example 6:

### Chemical characterization by scanning electron microscopy with X-ray detector (SEM/EDX) of the fibroin foams incubated in SBF

The fibroin foam samples were analysed in SEM/EDX in order to morphologically and compositionally identify the mineral formations constituted by calcium and phosphate salts. The results obtained are reported in Figure 3.

As already highlighted in Figure 1 (images C and F), there are two types of mineral deposits: one consisting of single crystals and one of crystalline aggregates of variable dimensions. While the single crystals are mainly composed of sodium (Na) and chlorine (CI), the agglomerates are mainly characterized by signals of calcium (Ca) and phosphorus (P) and, with lower intensity, magnesium (Mg). Note that the signals of carbon (C), oxygen (O), nitrogen (N), gold (Au) and palladium (Pd) are common to all spectra as they reflect the organic nature of the fibroin foam (C, O, N) and the sample preparation mode (Au/Pd sputtering).

### Example 7:

### Mineralization test of the fibroin foams incubated in the absence and in the presence of human mesenchymal stem cells

Human mesenchymal stem cells (MSCs) were purchased from Lonza and cultured in Dulbecco's Modified Eagle's Medium (DMEM), supplemented with 10% MSC Stimulatory Supplement, 100 U/mL penicillin-streptomycin and non-essential amino acids at 37°C in a fully humidified atmosphere at 5% CO₂. The fibroin foams were placed in a 96-well plate, immersed in 70% ethanol for 30 minutes, washed in phosphate buffered saline (PBS) three times, immersed in 100 U/mL penicillin-streptomycin overnight and washed three times in PBS. MSC (10⁴/50 µL) were seeded by pipetting onto different points of the foam to improve the distribution of the cells within the foam and allowed to adhere for 30 minutes. Subsequently, 150 µL of complete medium were added to each well. As a negative control, the fibroin foams were kept in the complete cell-free medium. The medium was changed every two days.

Calcium deposit on decellularized fibroin foams was revealed by von Kossa staining. The foams were washed twice with PBS (not containing magnesium/calcium ions) before cell lysis and protein extraction. Cell lysis was performed using the HEPES buffer (50 mM HEPES, pH 7.4, 150 mM NaCl, 10% (v/v) glycerol, 1% (v/v) Triton X-100, 1.5 mM MgCl₂, 1 mM EGTA) containing 100 mM NaF, 1 mM NaVO₄, 1 µg/mL leupeptin, and 1 µg/mL aprotinin. After 30 minutes on ice, the extracts were centrifuged for 10 minutes at 13,000g as cell lysate and stored at -20°C. The remaining deposited matrix was washed twice with PBS (not containing magnesium/calcium ions). To fix the matrix on the fibroin foams 25% formaldehyde was added in ultrapure water at room temperature for 10 minutes and then two washes with ultrapure water were performed. A 2.5% silver nitrate solution was added to the wells containing the fibroin foams and they were then placed under UV rays for 30 minutes. Finally, 5% sodium thiosulphate was used to remove excess unreacted silver.

The human mesenchymal stem cells (MSCs) were cultured for 7 days in the fibroin foams functionalized with the biomimetic peptides, using a non-functionalized fibroin foam as a reference. The cell-free foams were incubated for a similar time. The extent of calcium deposit was studied by means of von Kossa staining and quantified by image analysis with ImageJ software. Briefly, the images were converted to 16-bit greyscale images. The threshold was adjusted manually, and the percentage area covered by the signal above the threshold was measured.

As shown in Figure 4, the fibroin foams functionalized with the biomimetic peptides exhibit higher amounts of calcium deposits than the non-functionalized foam. The amount of calcium deposited by the action of biomimetic peptides is higher in the samples incubated in the presence of MSC than in those incubated without MSC. From the detailed description and from the above Examples, the advantages achieved by the peptides of the present invention are evident. In particular, these peptides have been shown to be surprisingly and advantageously suitable for use in bone, dental and periodontal regeneration, favouring a rapid and effective mineralization.

## Claims

1. Peptides consisting of the general formula (I):
Xaa1-Ser-Gly-Tyr-Glu-Tyr-Xaa2 (SEQ ID NO: 5) wherein:
- when Xaa1 is Val-Asn-Gly-Gly-Tyr (SEQ ID NO:6),
Xaa2 is Ala-Trp-Ser-Ser-Glu-Ser-Asp-Phe (SEQ ID NO:7)
or
Xaa2 is Ala-Trp;
- when Xaa1 is absent,
Xaa2 is Ala-Trp-Ser-Ser-Glu-Ser-Asp-Phe (SEQ ID NO:7);
and
- when Xaa1 is Gly-Pro-Tyr-Val-Ala-His-Gly-Gly-Tyr (SEQ ID NO:8),
Xaa2 is absent, wherein said peptides are chosen from the group consisting of: 
SEQ ID NO:1 Val-Asn-Gly-Gly-Tyr-Ser-Gly-Tyr-Glu-Tyr-Ala-Trp-Ser-Ser-Glu-Ser-Asp-
Phe,
SEQ ID NO:2 Ser-Gly-Tyr-Glu-Tyr-Ala-Trp-Ser-Ser-Glu-Ser-Asp-Phe,
SEQ ID NO:3 Gly-Pro-Tyr-Val-Ala-His-Gly-Gly-Tyr-Ser-Gly-Tyr-Glu-Tyr, and
SEQ ID NO:4 Val-Asn-Gly-Gly-Tyr-Ser-Gly-Tyr-Glu-Tyr-Ala-Trp.

2. Peptides according to claim 1, for use as a medicament.

3. Peptides according to any one of claims from 1 to 2, for use in bone, dental and periodontal regeneration.

4. The peptides for use according to claim 3, wherein said bone, dental and periodontal regeneration is in the treatment of a pathology of the bone, dental or periodontal tissue.

5. The peptides for use according to claim 4, wherein said pathology of the bone, dental or periodontal tissue is chosen from the group comprising: fractures, bone gaps, sarcoma, dentinal hypersensitivity, pyorrhea.

6. Composition comprising one or more peptides according to claim 1, their salts and excipients or additives acceptable in the biomedical, cosmetic or pharmaceutical field.

7. The composition according to claim 6, further comprising hyaluronic acid and / or its salts, chitosan, alginic acid, silk fibroin, propylene glycol, propylene glycol alginate, poloxamers, chondroitin sulfate, collagen, gelatin, elastin, polylactic acid (PLA), poly(lactic-co-glycolic) acid (PLGA), polyglycolic acid (PGA), polyvinyl alcohol (PVA), polycaprolactone (PCL), bioglasses, hydroxyapatite, calcium salts, decellularized bone matrix, pectin, sericin, cellulose, fibrin and their combinations.

8. The composition according to any one of claims 6 and 7, wherein said composition is in the form of gel, paste, foam, putty, powder, membrane, fabric, compact solid grain or film or is contained/adsorbed within micellar elements or particles, hollow fibers or nanofibers, membranes, ceramics or nanotubes, porous and/or trabecular metal structures, textile products, nanobubbles, sol-gels or their combinations.

9. The composition comprising one or more peptides according to claim 1, for use as a medicament.

10. The composition according to any one of claims from 6 to 8, for use in bone, dental and periodontal regeneration.

11. Use of the composition according to any one of claims from 6 to 8, for the functionalization of prostheses, screws, fixings, inserts or supports in metal, ceramic or natural or synthetic polymeric material.

12. Cosmetic use of the composition according to any one of claims from 6 to 8.

13. Use of the peptides according to claim 1, for the functionalization of prostheses, screws, fixings, inserts or supports made of metal, ceramic or natural or synthetic polymeric material.

## Patentansprüche

1. Peptide, bestehend aus der allgemeinen Formel (I):
Xaa1-Ser-Gly-Tyr-Glu-Tyr-Xaa2 (SEQ ID NO: 5) wobei:
- wenn Xaa1 Val-Asn-Gly-Gly-Tyr (SEQ ID NO:6) ist,
ist Xaa2 Ala-Trp-Ser-Ser-Glu-Ser-Asp-Phe (SEQ ID NO:7)
oder
ist Xaa2 Ala-Trp;
- wenn Xaa1 abwesend ist,
ist Xaa2 Ala-Trp-Ser-Ser-Glu-Ser-Asp-Phe (SEQ ID NO:7);
und
- wenn Xaa1 Gly-Pro-Tyr-Val-Ala-His-Gly-Gly-Tyr (SEQ ID NO:8) ist,
ist Xaa2 abwesend, wobei die Peptide aus der Gruppe ausgewählt sind, bestehend aus: 
SEQ ID NO:1 Val-Asn-Gly-Gly-Tyr-Ser-Gly-Tyr-Glu-Tyr-Ala-Trp-Ser-Ser-Glu-
Ser-Asp- Phe,
SEQ ID NO:2 Ser-Gly-Tyr-Glu-Tyr-Ala-Trp-Ser-Ser-Glu-Ser-Asp-Phe,
SEQ ID NO:3 Gly-Pro-Tyr-Val-Ala-His-Gly-Gly-Tyr-Ser-Gly-Tyr-Glu-Tyr, und
SEQ ID NO: 4 Val-Asn-Gly-Gly-Tyr-Ser-Gly-Tyr-Glu-Tyr-Ala-Trp.

2. Peptide nach Anspruch 1 zur Verwendung als Medikament.

3. Peptide nach einem der Ansprüche 1 bis 2 zur Verwendung bei der Knochen-, Zahn- und Zahnfleischregeneration.

4. Peptide zur Verwendung nach Anspruch 3, wobei die Knochen-, Zahn- und Zahnfleischregeneration bei der Behandlung einer Pathologie des Knochens, Zahns oder Zahnfleischgewebes erfolgt.

5. Peptide zur Verwendung nach Anspruch 4, wobei die Pathologie des Knochens, Zahns oder Zahnfleischgewebes ausgewählt ist aus der Gruppe, umfassend: Frakturen, Knochenspalte, Sarkom, Dentinüberempfindlichkeit, Parodontitis.

6. Zusammensetzung, umfassend ein oder mehrere Peptide nach Anspruch 1, deren Salze und Hilfsstoffe oder Zusatzstoffe, die im biomedizinischen, kosmetischen und pharmazeutischen Bereich verträglich sind.

7. Zusammensetzung nach Anspruch 6, ferner umfassend Hyaluronsäure und/oder deren Salze, Chitosan, Alginsäure, Seidenfibroin, Propylenglykol, Propylenglykolalginat, Poloxamere, Chondroitinsulfat, Kollagen, Gelatine, Elastin, Polymilchsäure (PLA), Poly(milch-co-glykol)säure (PLGA), Polyglykolsäure (PGA), Polyvinylalkohol (PVA), Polycaprolacton (PCL), Bioglas, Hydroxylapatit, Kalziumsalze, dezellularisierte Knochenmatrix, Pektin, Sericin, Zellulose, Fibrin und deren Kombinationen.

8. Zusammensetzung nach einem der Ansprüche 6 und 7, wobei die Zusammensetzung in Form von Gel, Paste, Schaum, Knete, Pulver, Membran, Gewebe, kompaktem festen Korn oder Film vorliegt oder in mizellaren Elementen oder Partikeln, Hohlfasern oder Nanofasern, Membranen, Keramiken oder Nanoröhren, porösen und/oder trabekulären Metallstrukturen, Textilprodukten, Nanoblasen, Sol-Gelen oder deren Kombinationen enthalten/adsorbiert ist.

9. Zusammensetzung, umfassend ein oder mehrere Peptide nach Anspruch 1, zur Verwendung als Medikament.

10. Zusammensetzung nach einem der Ansprüche 6 bis 8 zur Verwendung bei der Knochen-, Zahn- und Zahnfleischregeneration.

11. Verwendung der Zusammensetzung nach einem der Ansprüche 6 bis 8 zur Funktionalisierung von Prothesen, Schrauben, Befestigungen, Einsätzen oder Stützen aus Metall, Keramik oder natürlichem oder synthetischem Polymermaterial.

12. Kosmetische Verwendung der Zusammensetzung nach einem der Ansprüche 6 bis 8.

13. Verwendung der Peptide nach Anspruch 1 zur Funktionalisierung von Prothesen, Schrauben, Befestigungen, Einsätze oder Stützen aus Metall, Keramik oder natürlichem oder synthetischem Polymermaterial.

## Revendications

1. Peptides répondant à la formule générale (I) :
Xaa1-Ser-Gly-Tyr-Glu-Tyr-Xaa2 (SEQ ID NO: 5)
dans laquelle :
- lorsque Xaa1 est Val-Asn-Gly-Gly-Tyr (SEQ ID NO:6),
Xaa2 est Ala-Trp-Ser-Ser-Glu-Ser-Asp-Phe (SEQ ID NO:7)
ou
Xaa2 est Ala-Trp ;
- lorsque Xaa1 est absent,
Xaa2 est Ala-Trp-Ser-Ser-Glu-Ser-Asp-Phe (SEQ ID NO:7) ;
et
- lorsque Xaa1 est Gly-Pro-Tyr-Val-Ala-His-Gly-Gly-Tyr (SEQ ID NO:8), Xaa2 est absent, lesdits peptides étant choisis dans le groupe constitué de : 
SEQ ID NO:1 Val-Asn-Gly-Gly-Tyr-Ser-Gly-Tyr-Glu-Tyr-Ala-Trp-Ser-Ser-Glu-
Ser-Asp- Phe,
SEQ ID NO:2 Ser-Gly-Tyr-Glu-Tyr-Ala-Trp-Ser-Ser-Glu-Ser-Asp-Phe,
SEQ ID NO:3 Gly-Pro-Tyr-Val-Ala-His-Gly-Gly-Tyr-Ser-Gly-Tyr-Glu-Tyr, et
SEQ ID NO:4 Val-Asn-Gly-Gly-Tyr-Ser-Gly-Tyr-Glu-Tyr-Ala-Trp.

2. Peptides selon la revendication 1, pour une utilisation comme médicament.

3. Peptides selon l'une quelconque des revendications 1 à 2, pour une utilisation dans la régénération osseuse, dentaire et parodontale.

4. Peptides pour une utilisation selon la revendication 3, dans lesquels ladite régénération osseuse, dentaire et parodontale est dans le traitement d'une pathologie du tissu osseux, dentaire ou parodontal.

5. Peptides pour une utilisation selon la revendication 4, dans lesquels ladite pathologie du tissu osseux, dentaire ou parodontal est choisie dans le groupe comprenant : les fractures, les écarts osseux, le sarcome, l'hypersensibilité dentinaire, la pyorrhée.

6. Composition comprenant un ou plusieurs peptides selon la revendication 1, leurs sels et excipients ou additifs acceptables dans le domaine biomédical, cosmétique ou pharmaceutique.

7. Composition selon la revendication 6, comprenant en outre de l'acide hyaluronique et / ou ses sels, du chitosane, de l'acide alginique, de la fibroïne de soie, du propylène glycol, de l'alginate de propylène glycol, des poloxamères, du sulfate de chondroïtine, du collagène, de la gélatine, de l'élastine, de l'acide polylactique (PLA), de l'acide poly (lactique-co-glycolique) (PLGA), de l'acide polyglycolique (PGA), de l'alcool polyvinylique (PVA), de la polycaprolactone (PCL), des bioverres, de l'hydroxyapatite, des sels de calcium, de la matrice osseuse décellularisée, de la pectine, de la séricine, de la cellulose, de la fibrine et de leurs combinaisons.

8. Composition selon l'une quelconque des revendications 6 et 7, dans laquelle ladite composition est sous forme de gel, de pâte, de mousse, de mastic, de poudre, de membrane, de tissu, de grain ou de film solide compact ou est contenue/adsorbée dans des éléments ou des particules micellaires, des fibres creuses ou des nanofibres, des membranes, des céramiques ou des nanotubes, des structures métalliques poreuses et/ou trabéculaires, des produits textiles, des nanobulles, des sol-gels ou leurs combinaisons.

9. Composition comprenant un ou plusieurs peptides selon la revendication 1, pour une utilisation comme médicament.

10. Composition selon l'une quelconque des revendications 6 à 8, pour une utilisation dans la régénération osseuse, dentaire et parodontale.

11. Utilisation de la composition selon l'une quelconque des revendications 6 à 8, pour la fonctionnalisation de prothèses, vis, fixations, inserts ou supports en métal, céramique ou matériau polymère naturel ou synthétique.

12. Utilisation cosmétique de la composition selon l'une quelconque des revendications 6 à 8.

13. Utilisation des peptides selon la revendication 1, pour la fonctionnalisation de prothèses, vis, fixations, inserts ou supports en métal, céramique ou matériau polymère naturel ou synthétique.
